# EUROPEAN PATENT APPLICATION

(11) **EP 1 614 463 A1**
(43) Date of publication of application: **11.01.2006**
(21) Application number: 04380102.6
(22) Date of filing: 05.05.2004
(51) Int. Cl.: B01J 35/00, B01J 23/34, B01J 21/16, B01J 27/135, A61L 9/20

(54) **Photocatalytic procedure for the control of macrobiota in the air in indoor environments**

(30) Priority: 30.01.2004 ES 200400199
(71) Applicant: Envirocontrol S.A, 28033 Madrid (ES)
(72) Inventor: Cuartero Sanchez, Luis Envirocontrol S.A., 28033 Madrid (ES)
(74) Representative: Ballesteros, Pedro Gonzales

(57) **Abstract**

Photocatalytic procedure for the control of microbiota in indoor environments, that consists of passing the air over a catalyst obtainable by the following method:
A base material such as magnesium silicate, a semiconductor and an oxidizer such as permanganate, periodic acid or periodate are mixed with water to form a paste, extruded to a honeycomb shape, dried and calcinated.

## Description

### OBJECT OF THE INVENTION

The present descriptive report refers to a Patent application for an Invention relating to a photocatalytic procedure for the control of microbiota in indoor environments, that consists of passing the air of an area, characterised by a microbiological burden, through a surface impregnated and photo-activated with ultraviolet UV-C photons, said surface onto which the characteristic microbiota is deposited achieving its photo-oxidation in air to CO₂, by total oxidation of the organic constituent matter of these cells.

### FIELD OF THE INVENTION

This invention has its application within the industry dedicated to the manufacture of apparatus, devices and auxiliary elements for air cleaning: buildings, houses, hospitals, schools, nursery schools, pharmaceutical industry, car industry, farms, restaurants, food industry, confectionery shops, electronics, hydroponics etc.

### BACKGROUND OF THE INVENTION

The problem of the poor quality of air within buildings dates from the middle of the decade of the 70s, as a consequence of the architectural modifications imposed by energy saving measures and high oil prices.
The glazing of modern buildings prevents in many cases the opening of windows precluding natural renewal of air.
Often the lack of ventilation in interior spaces leads to dependency on mechanical air conditioning systems that, by increasing the air recirculation up to 70 %, give rise to an increase of contaminants and deterioration of the quality of the indoor air.

To this it is necessary to add the current life-habits that lead to a great part of the population spending almost 90 % of their time in closed spaces.

Although the contaminants present in air can be of a very varied nature, it is necessary to emphasize biological contaminants that are found in the air in the form of bioaerosols; that is, as agents suspended in the air, whether isolated or contained in small drops of water.

The composition of the aerosols is varied: microorganisms such as bacteria, fungi, virus or protozoan, and products of the metabolism or of the growth of microorganisms, such as micro-toxins or endotoxins.

These circumstances and this type of agent have brought about types of patology that are commonly differentiated into two groups: the so-called sick-building syndrome, and disease inherent in the building (pathologies such as legionelosis or other respiratory infections, hypersensitivity reactions, allergies, etc.)

The social concern produced by the appearance of the aforementioned illnesses or others, attributed to different air pollutants, makes it essential to know the microbiological quality of the confined air, as well as to establish mechanisms of prevention.

Although the total absence of microorganisms in indoors environments is completely impossible, it is vital to control the levels of contamination in order that certain thresholds are not exceeded.

The applicant does not know of the existence at present of a photocatalytic procedure for the control of microbiota in indoor environments.

### DESCRIPTION OF THE INVENTION

The photocatalytic procedure for the control of the microbiota in the air in indoor environments, that the invention proposes, forms in itself an evident innovation within its specific area of application.

More specifically, the photocatalytic procedure for the control of microbiota in indoor environments consists in passing the air from an area characterised by a microbiological load, through an impregnated surface photo-activated with ultraviolet UV-C photons. It is primarily based in that the surface is formed by a base that contains one or several catalyst semiconductor materials both it and the base and the semiconductors are impregnated with a highly oxidizing substance.

The base is natural magnesium silicate and water, with suitable rheological properties to present the product in a granulated form, or to extrusion mould monoliths with square section channels (honeycomb type); it also has the advantage of being indifferent to the reaction medium, of achieving good adhesion with the semiconductor materials and oxidizing substances, achieving a minimum decrease of the catalyzer activity; the semiconductor catalysts are TiO₂, ZnO; Al₂O₃, MgO, SiO₂, Cds, Fe₂O₃, ZnS, FeOOH, and the impregnations are KMnO₄, periodic acid, and periodate; photoactivation is carried out by photons principally short wavelength UV-C (200-295 nm) ultraviolet, and it is where there is more germicidal effect, the optimum UV germicidal action occurs at 265 nm, genetic material or DNA being the target for the UV, since the UV light penetrates the cell wall and cytoplasmatic membrane, it causes a molecular restructuring of the microorganism DNA that in this way prevents it from reproducing; if a cell cannot reproduce, it is considered dead.

Electron transfer processes occur at the surface of the solid as a consequence of this photo activation, the valency electrons can be excited to the conduction band creating highly reactive electron - hole pairs, the excess electrons in the conduction band react with the molecular oxygen to form superoxide ions that can form hydroxyl radicals, the surface of the holes reacts with the absorbed water or with the OH - groups similarly to form hydroxyl radicals, in this way the photoactivated surface is capable of promoting catalytically photo-assisted reactions.

### PREFERABLE EMBODIMENT OF THE INVENTION

The photocatalytic procedure for the control of microbiota in indoor environments that is proposed, is primarily based on the extrusion moulding of monoliths with square section channels (honeycomb type, but of square section) of between 2 and 4 mm side, initially pastes of mixtures of semiconductor catalyst (10-50 % by weight), base (40-90 %) and oxidizer (1-20 %) are prepared, the consistency and plasticity of the paste is made suitable by controlling the quantity of water added, the piece later extruded is cut to the suitable size and carefully dried (80°-100° for 4-8 hours with a heating rate of rise of 3°-5°C min⁻¹), to avoid modifications in the geometry.

Once dry, it undergoes a suitable thermal treatment to give it mechanical stability (200 - 500°C for 2-4 hours), compound materials have greater porosity than those obtained from pure components, by virtue of their fibrillose structure; for the granulated surface the method previously described can be used without the need to extrude the mixture, and controlling the quantity of water incorporated so that the final product obtained has a % of moisture between 5-10 %.

Ultraviolet lamps can differ in geometry, power, life-time and type, optical fibers have the advantages of a direct transfer of radiation to the surface and of high surface area activated, in relation with the reactor volume.

To summarize, the invention is devised from a base formed of natural magnesium silicates with rheological properties suitable to obtain a final product in granulated form or to extrusion mould monoliths, or for impregnations with a 40 %-90 % ratio of the total weight and at the same time incorporating single-compound catalyst semiconductors or a mixture of them with a ratio of 10 %-50 % of total weight, preferably TiO₂, ZnO, Al₂O₃, MgO, SiO₂, CdS, Fe₂O₃, ZnS, FeOOH, these catalyzing semiconductors being capable of promoting catalytically photo-assisted reactions in which electron-transfer processes take place.

The invention includes impregnations of solutions of oxidizing substances formed by KMnO₄, periodic acid and periodate in a ratio that ranges between 1 % and 20 % of the total weight, photo-activation is carried out by photons, mainly short wavelength UV-C (200-295 nm) ultraviolet.

## Claims

1. Photocatalytic procedure for the control of microbiota in indoor environments, **characterised by** being based on the extrusion moulding of monoliths with honeycomb type square section channels, that have dimensions that range between 2 mm and 4 mm per side; mixtures of semiconductor catalyst pastes, 10-50 % by weight, being prepared; a base between 40 % and 90 % incorporated, and an oxidizer between a 1 % and 20 % also incorporated; the consistency and plasticity of the paste obtained being made suitable by controlling the quantity of water added, the extruded piece being later cut and dried at a temperature that ranges between 80° and 100° centigrade for a period of 4 to 8 hours, with a heating rate of rise of 3 ° to 5 ° centigrade min⁻¹, and once dry it is subjected to a suitable thermal treatment to provide mechanical stability, between 200° centigrade and 500° centigrade for a time that ranges between 2 and 4 hours.

2. Photocatalytic procedure for the control of microbiota in indoor environments, according to the first claim, **characterised in that** for the granulated surface we can use the method previously described without the need to extrude the mixture and controlling the quantity of water added so that the final product obtained has a percentage of moisture between 5-10 %.

3. Photocatalytic procedure for the control of microbiota in indoor environments, according to the first claim, **characterised in that** the base is formed by natural silicates of magnesium.

4. Photo-catalytic procedure for the control of microbiota in indoor environments, according to the first claim, **characterised in that** the catalyst semiconductors can be formed by a single compound, or by a mixture with each other of TiO₂, ZnO, Al₂O₃, MgO, SiO₂, CdS, Fe₂O₃, ZnS y FeOOH.

5. Photo-catalytic procedure for the control of microbiota in indoor environments, according to the first claim, **characterised in that** the impregnations of solutions of oxidizing substances are formed of KMnO₄, periodic acid and periodate.

6. Photo-catalytic procedure for the control of microbiota in indoor environments, according to the first claim, **characterised in that** the photoactivation is carried out by photons principally short wavelength UV-C (200-295 nm) ultraviolet.
